(19) Europäisches Patentamt
European Patent Office
Office européen des brevets

(11) Veröffentlichungsnummer: **0 355 383**
**A1**

(12) # EUROPÄISCHE PATENTANMELDUNG

(21) Anmeldenummer: **89113003.1**

(22) Anmeldetag: **15.07.89**

(51) Int. Cl.$^4$: **C07F 1/08 , C07D 215/30 , //A01N55/02**

(30) Priorität: **28.07.88 CH 2881/88**

(43) Veröffentlichungstag der Anmeldung: **28.02.90 Patentblatt 90/09**

(84) Benannte Vertragsstaaten: **AT CH DE ES FR GB IT LI SE**

(71) Anmelder: **F. HOFFMANN-LA ROCHE AG**
**Postfach 3255**
**CH-4002 Basel(CH)**

(72) Erfinder: **Hugener, Hans Peter**
**Buenstrasse 52**
**CH-8600 Dübendorf(CH)**
Erfinder: **Roth, Willy**
**Verstorben**
**Verstorben(CH)**

(74) Vertreter: **Aschert, Hubert et al**
**Grenzacherstrasse 124 Postfach 3255**
**CH-4002 Basel(CH)**

(54) **Solubilisierungsverfahren.**

(57) Es wird ein Solubilisierungsverfahren zur Herstellung eines in organischen Lösungsmitteln löslichen Kupferoxinat-Präparates bzw. einer Kupferoxinatlösung unter Verwendung bestimmter Phosphorsäureester als Lösungsvermittler beschrieben.

EP 0 355 383 A1

Xerox Copy Centre

## Solubilisierungsverfahren

Die vorliegende Erfindung betrifft ein Solubilisierungsverfahren, und zwar ein Verfahren zur Herstellung eines in organischen Lösungsmitteln löslichen Kupferoxinat-Präparates bzw. einer Kupferoxinatlösung in einem organischen Lösungsmittel.

Kupferoxinat (oxine-copper), d.h. der Komplex von Cu (II) mit 8-Hydroxychinolin, sowie dessen fungizide Eigenschaften sind seit langem bekannt und in zahlreichen Publikationen beschrieben (z.B. Pesticide Manual, 8 Ed., Seite 622). Es ist auch bekannt, dass Kupferoxinat unter stark sauren Bedingungen (pH <2,7) wasserlöslich ist. Für bestimmte Anwendungen ist es jedoch vorteilhaft bzw. notwendig, Lösungen von Kupferoxinat in einem organischen Lösungsmittel zur Verfügung zu haben. Derartige Lösungen wurden bisher dadurch hergestellt, dass man Kupferoxinat mit Nonylphenol bzw. 2-Aethylhexansaure-Ni-Salz solubilisierte (U.S. Patentschriften 2,651,379, 2,561,380 und 2,608,556). Nickelsalze und auch Nonylphenol gehören jedoch zu den toxikologisch bedenklichen Stoffen, welche aus diesem Grunde in gewissen Ländern nicht mehr verwendet werden dürfen.

Es wurde nun überraschenderweise gefunden, dass man Kupferoxinat unter Verwendung von bestimmten Phosphorsäureestern als Lösungsvermittler in organischen Lösungsmitteln auflösen kann.

Bei den hierbei als Lösungsvermittler verwendbaren Phosphorsäureestern handelt es sich um flüssige Substanzen, welche der Formel

```
      O
      ||
   RO-P-OR'        (I)
      |
      OH
```

entsprechenden,
worin R und R′ Wasserstoff oder einen Alkylrest mit bis zu 13 C-Atomen darstellt, wobei zumindest eines der Symbole R und R′ ein Alkylrest ist.

Bei den Verbindungen der obigen Formel I handelt es sich somit um bekannte Mono- und Diester der Phosphorsäure.

Das erfindungsgemässe Verfahren zur Herstellung eines in organischen Lösungsmitteln löslichen Kupferoxinat-Präparates bzw. einer Kupferoxinatlösung in einem organischen Lösungsmittel ist somit dadurch gekennzeichnet, dass man als Lösungsvermittler eine Verbindung der obigen allgemeinen Formel I, worin R und R′ die obige Bedeutung haben, verwendet.

Die Alkylgruppen R und R′ können geradkettig oder verzweigt sein, wobei geradkettige Alkylgruppen 1-8 C-Atome enthalten können, verzweigte Alkylgruppen jedoch bis zu 13 C-Atomen also 3-13 C-Atome. Beispiele solcher Alkylgruppen sind Methyl, Aethyl, Propyl, Isopropyl, Butyl, Isobutyl, Amyl, Hexyl, 2-Aethyl hexyl, Octyl, Isooctyl und Isotri decyl.

Als Lösungsvermittler können auch Gemische von Mono- und Diestern der obigen Formel I verwendet werden.

Besonders bevorzugte Lösungsvermittler der obigen Formel I sind Mono- bzw. Diester der Phosphporsäure mit 2-Aethyl-hexyl bzw. Gemische solcher Mono- und Diester.

Das Gewichtsverhältnis zwischen Kupferoxinat und Lösungsvermittler kann innerhalb eines breiten Bereiches variieren.

Zweckmässig verwendet man Kupferoxinat und Lösungsvermittler in einem Gewichtsverhältnis von etwa 1:0,5 bis etwa 1:50, zweckmässig etwa 1:1 bis etwa 1:50, und vorzugsweise in einem Gewichtsverhältnis von etwa 1:4 bis 1:15. Ein Gewichtsverhältnisbereich von etwa 1:6 bis etwa 1:10 ist besonders bevorzugt.

Durch Vermischen des Kupferoxinats mit dem Lösungsvermittler erhält man ein Präparat, welches zur Herstellung einer Kupferoxinat Lösung in einem organischen Lösungsmittel geeignet ist. Zur Herstellung einer derartigen Lösung kann man entweder so vorgehen, dass man das durch Vermischen des Kupferoxinats mit dem Lösungsvermittler erhaltene Gemisch mit dem Lösungsmittel verdünnt, oder dass man das Kupferoxinat, den Lösungsvermittler und das Lösungsmittel, gegebenenfalls unter Erwärmen, miteinander

vermischt. Durch das Erwärmen (je nach Lösungsmittel beispielsweise auf Temperaturen zwischen etwa 50° C und 120° C) wird der Löseprozess beschleunigt.

Man kann auf diese Weise Lösungen von Kupferoxinat mit sehr verschiedenen Kupferoxinatgehalten, bis etwa 15% Kupferoxinat, herstellen. Bevorzugte Lösungen sind solche mit einem Gehalt zwischen etwa 1 und 10 Gew.-%.

Als Lösungsmittel kommen die für ähnliche Produkte üblichen technischen Lösungsmittel in Frage, also beispiels weise aliphatische und aromatische Kohlenwasserstoffe, Ketone, Ester, Lactone und dergleichen. Beispiele solcher Lösungsmittel sind das aus Alkylbenzolen bestehende SOLVESSO® 100, das aus Alkylnaphthalinen bestehende SOLVESSO® 200, das aus aliphatischen und aromatischen Kohlenwasserstoffen bestehende Sangajol, Benzylalkohol, Methylisobutylketon, γ-Butyroiaoton und Propylenglykolmonomerhyläther (DOWANOL® PMA).

Die gemäss dem vorliegenden Verfahren hergestellten Lösungen können direkt als gebrauchsfertige Lösungen oder nach Verdünnung mit Lösungsmitteln, vorzugsweise Mineralölen, gemäss den üblichen Applikationsmethoden, wie z.B. Tauchen. Sprüchen, Streichen usw. angewandt werden. Durch Zusatz von Emulgatoren zu den erfindungsgemäss erhaltenden Lösungen erhält man emulgierbare Konzentrate, welche nach Verdünnung mit Wasser appliziert werden können.

## Beispiel 1

Durch Verrühren von 10 Teilen Kupferoxinat, 60 Teilen eines etwa äquimolaren Gemisches von Mono-2-äthyl-hexyl-phosphat, Di-2-äthyl-hexyl-phosphat und 30 Teilen Aethylenglykolphenyläther bei 80° C erhält man eine grünblaue Lösung von Kupferoxinat.

## Beispiel 2

Durch Verrühren eines Gemisches von 8 Teilen Kupferoxinat, 60 Teilen einer etwa äquimolaren Mischung der in Beispiel 1 genannten Phosphate und 32 Teilen Benzylalkohol während 2 Stunden bei 120° C erhält man eine tief-blaugrüne Lösung von Kupferoxinat.

## Beispiel 3

Es werden 2 Teile Kupferoxinat, 45 Teile des Monobutyl-phosphorsäureesters, 33 Teile Solvesso 100 (Alkylbenzolgemisch) und 20 Teile Nonylphenolpolyäthoxylat bei Raumtemperatur etwa 1 Stunde gerührt bis die gesamte Menge des Kupferoxinats gelöst ist. Es entsteht hierbei eine grünliche, durchsichtige Lösung, welche, aufgrund des Emulgatorgehaltes (Nonylphenolpolyäthoxylat), mit Wasser verdünnbar ist.

In den nachstehenden Tabellen A-C ist die Zusammensetzung verschiedener Kupferoxinatpräparate gemäss der vorliegenden Erfindung dargestellt, wobei die verwendeten Kurzbezeichnungen die folgenden Bedeutungen haben:

MDM: Gemisch aus Mono- und Dimethylester der Phosphorsäure
MDB: Gemisch aus Mono- und Dibutylester der Phosphorsäure
MDAH: Gemisch aus Mono- und Di-2-ätyhl-hexylester der Phosphorsäure
MDIT: Gemisch aus Mono- und Diisotridecylester
MOB: Monobutylester der Phosphorsäure

Diese Produkte werden u.a. von der Firma HOECHST hergestellt.

Die Tabelle A zeigt die Anwendung verschiedener Lösungsvermittler bei Verwendung eines Kupferoxinat/Lösungsmittel (Cyclohexanon)-Verhältnises von 1:10.

Tabelle A

| Zusammensetzung in Gew.-% | | | | | |
|---|---|---|---|---|---|
| Kupferoxinat | 5 | 5 | 5 | 5 | 5 |
| Lösungsvermittler MDM | 45 | | | | |
| " MDB | | 45 | | | |
| " MOB | | | 45 | | |
| " MDAH | | | | 45 | |
| " MDIT | | | | | 45 |
| Cyclohexanon | 50 | 50 | 50 | 50 | 50 |

Die Solubilisierung erfolgt durch 1-stündiges Erhitzen auf 120 °C. Man erhält tiefgrüne Lösungen, die zur Herstellung von Kupferoxinatlösungen in organischen Lösungsmitteln geeignet sind.

Die Tabelle B zeigt die Verwendung verschiedener Lösungsmittel.

Tabelle B

| Zusammensetzung in Gew.-% | | | | | |
|---|---|---|---|---|---|
| Kupferoxinat | 5 | 8 | 10 | 5 | 5 |
| Lösungsvermittler MDM | 45 | | | | |
| " MDAH | | 60 | 60 | 45 | |
| " MDIT | | | | | 45 |
| Propylenglycolmonomethyläther | 50 | | | | |
| Benzylalkohol | | 32 | | | |
| Aethylenglycolphenyläther | | | 30 | | |
| EXSOL® D-100 (EXXON) | | | | 50 | |
| SOLVESSO® 200 (EXXON) | | | | | 50 |

Die Solubilisierung wird hier ebenfalls durch 1- bis 2-stündige Erwärmung auf 50 bis 120 °C erreicht.

Die Tabelle C zeigt Beispiele für verschiedene Konzentrationen und Gewichtsverhältnisse Kupferoxinat:Lösungsvermittler, wobei sich die letzte Spalte auf ein in Wasser emulgierbares Präparat bezieht.

Tabelle C

| Zusammensetzung in Gew.-% | | | | |
|---|---|---|---|---|
| Kupferoxinat | 4 | 7 | 15 | 2 |
| Lösungsvermittler MOB | 2 | | 65 | 45 |
| " MDAH | | 14 | | |
| Benzylalkohol | 94 | 79 | 20 | |
| SOLVESSO® 100 (EXXON) | | | | 33 |
| Nonylphenolpolyäthoxylat (Emulgator) | | | | 20 |
| Verhältnis Kupferoxinat: | | | | |
| Lösungsvermittler | 1:1 | 1:2 | 1:4 | 1:22 |
| Solubilisierungsbedingungen: | 7h/120 °C | 1h/120 °C | 1h/120 °C | 1h/RT |

**Ansprüche**

1. Verfahren zur Herstellung eines in organischen Lösungsmitteln löslichen Kupferoxinat-Präparates

bzw. einer Kupferoxinatlösung in einem organischen Lösungsmittel, dadurch gekennzeichnet, dass man als Lösungsvermittler eine Verbindung der allgemeinen Formel

$$\begin{array}{c} O \\ \| \\ RO-P-OR' \\ | \\ OH \end{array} \qquad (I)$$

verwendet,
worin R und R′ Wasserstoff oder eine Alkylgruppe mit bis zu 13 Kohlenstoffatomen darstellt, wobei zumindest eines der Symbole R und R′ eine Alkylgruppe ist.

2. Verfahren nach Anspruch 1, dadurch gekennzeichnet, dass man als Verbindung der Formel I eine solche verwendet, worin R und/oder R′ eine geradkettige Alkylgruppe mit 1-8 Kohlenstoffatomen bedeutet.

3. Verfahren nach Anspruch 1, dadurch gekennzeichnet, dass man als Verbindung der Formel I eine solche verwendet, worin R und/oder R′ eine verzweigtkettige Alkylgruppe mit 3-13 Kohlenstoffatomen bedeutet.

4. Verfahren nach Anspruch 1 oder 3, dadurch gekennzeichnet, dass man als Verbindung der Formel I eine solche ver wendet, worin R und/oder R′ 2-Aethyl-hexyl bedeutet.

5. Verfahren nach einem der Ansprüche 1-4, dadurch gekennzeichnet, dass man als Lösungsvermittler ein Gemisch von Mono- und Diestern der Formel I verwendet.

6. Verfahren nach einem der Ansprüche 1-5, dadurch gekennzeichnet, dass man Kupferoxinat und Lösungsvermittler in einem Gewichtsverhältnis von 1:0,5 bis 1:50, zweckmässig von 1:1 bis 1:50, verwendet.

7. Verfahren nach einem der Ansprüche 1-6, dadurch gekennzeichnet, dass man Kupferoxinat und Lösungsvermittler in einem Gewichtsverhältnis von etwa 1:4 bis 1:15 verwendet.

8. Verfahren nach einem der Ansprüche 1-7, dadurch gekennzeichnet, dass man Kupferoxinat und Lösungsvermittler in einem Gewichtsverhältnis von etwa 1:6 bis etwa 1:10 verwendet.

9. Verfahren nach Anspruch 1, zur Herstellung einer Kupferoxinatlösung in einem organischen Lösungsmittel, dadurch gekennzeichnet, dass man das durch Vermischen des Kupferoxinats mit dem Lösungsvermittler erhaltene Gemisch mit dem Lösungsmittel verdünnt. oder dass man das Kupferoxinat, den Lösungsvermittler und das Lösungsmittel, gegebenenfalls unter Erwärmen, miteinander vermischt.

Europäisches Patentamt

# EUROPÄISCHER RECHERCHENBERICHT

Nummer der Anmeldung

EP 89 11 3003

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 033 865 (G.D. FRONMULLER et al.)<br>* Spalte 2, Zeilen 24-32; Anspruch 1 *<br>--- | 1 | C 07 F 1/08<br>C 07 D 215/30 //<br>A 01 N 55/02 |
| A | US-A-2 755 280 (R. FEIGIN et al.)<br>* Anspruch 1 *<br>--- | 1 | |
| A | CHEMICAL ABSTRACTS Band 100, Nr. 18, 30. April 1984, Seite 706, Spalte 1, Zusammenfassung Nr. 150088g, Columbus, Ohio, USA; N.D. IORDANOV et al.: "Studies on the intermolecular interactions of metal chelate complexes VII. An EPR study of the CuL'2 + CuL''2 interactions in solution" & Izv. Khim. 1983, Band 16, Nr. 1,2,Seiten 214-223<br>--- | 1 | |
| A | CHEMICAL ABSTRACTS Band 68, Nr. 4, 22. Januar 1968, Seite 1596, Spalte 1, Zusammenfassung Nr. 16567e, Columbus, Ohio, USA; F.G. ZHAROVSKII et al.: "Solubilty of some 8-hydroxyquinolinates in organic solvents" & Zh. Anal. Khim. 1967, Band 22, Nr. 8, Seiten 1142-1145<br>--- | 1 | **RECHERCHIERTE SACHGEBIETE (Int. Cl.5)**<br>C 07 F 1/08<br>C 07 D 215/30<br>A 01 N 55/02 |
| D,A | US-A-2 608 556 (V.N. KALBERG)<br>* Ansprüche 7-17 *<br>----- | 1 | |

**Der vorliegende Recherchenbericht wurde für alle Patentansprüche erstellt**

| Recherchenort | Abschlußdatum der Recherche | Prüfer |
|---|---|---|
| BERLIN | 04-09-1989 | KAPTEYN H G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)